# DEMANDE DE BREVET EUROPEEN

(11) **EP 1 637 124 A1**
(43) Date de publication de la demande: **22.03.2006**
(21) Numéro de dépôt: 05291810.9
(22) Date de dépôt: 31.08.2005
(51) Int. Cl.: A61K 8/67, A61K 8/88, A61Q 19/00

(54) **Utilisation cosmétique d'une composition renfermant un dérivé d'acide ascorbique et des particules de polyamide**

(30) Priorité: 16.09.2004 FR 0452074
(71) Demandeur: L'OREAL, 75008 Paris (FR)
(72) Inventeur: Marion, Catherine, 92160 Antony (FR)
(74) Mandataire: Renard, Emmanuelle

(57) **Abrégé**

La présente invention se rapporte à l'utilisation cosmétique pour le soin des peaux grasses d'une composition renfermant, dans un milieu physiologiquement acceptable : (a) au moins un dérivé hydrosoluble d'acide ascorbique et (b) des particules poreuses de polyamide ayant un diamètre moyen, en volume, inférieur ou égal à 10 µm, étant entendu que la composition ne se trouve pas sous la forme d'une émulsion eau-dans-huile.

Elle se rapporte également à un procédé cosmétique de soin d'une peau grasse, en particulier en vue de matifier la peau, comprenant l'application topique sur celle-ci d'une composition telle que définie ci-dessus.

## Description

La présente invention se rapporte à l'utilisation cosmétique pour le soin des peaux grasses d'une composition renfermant, dans un milieu physiologiquement acceptable : (a) au moins un dérivé hydrosoluble d'acide ascorbique et (b) des particules poreuses de polyamide ayant un diamètre moyen, en volume, inférieur ou égal à 10 µm, étant entendu que la composition ne se trouve pas sous la forme d'une émulsion eau-dans-huile.

Elle se rapporte également à un procédé cosmétique de soin d'une peau grasse, en particulier en vue de matifier la peau, comprenant l'application topique sur celle-ci d'une composition telle que définie ci-dessus.

La brillance de la peau est un problème affectant plus particulièrement les adolescents mais qui peut aussi se manifester à l'âge adulte sous l'effet notamment d'une hyperproduction d'androgènes. Une peau brillante est généralement une peau hyper-séborrhéique caractérisée par une sécrétion et une excrétion exagérées de sébum conduisant généralement à un taux de sébum supérieur à 200 µg/cm² mesuré au niveau du front.

Le sébum est le produit naturel de la glande sébacée qui constitue une annexe de l'unité pilosébacée. Il s'agit essentiellement d'un mélange plus ou moins complexe de lipides. Classiquement, la glande sébacée produit du squalène, des triglycérides, des cires aliphatiques des cires de cholestérol et, éventuellement du cholestérol libre (Stewart,M.E., *Semin Dermatol* **11,** 100-105 (1992)). L'action des lipases bactériennes convertit une part variable des triglycérides formés en acides gras libres.

Le sébocyte constitue la cellule compétente de la glande sébacée. La production de sébum est associée à un programme de différenciation terminale de cette cellule. Durant cette différenciation, l'activité métabolique du sébocyte est essentiellement axée sur la biosynthèse des lipides *(la lipogenèse)* et plus précisément sur la néosynthèse d'acide gras.

Si le sébum constitue normalement un hydratant naturel de l'épiderme, la surproduction de sébum peut entraîner des désagréments esthétiques (peau luisante, moins bonne tenue du maquillage, formation de comédons), voire constituer un terrain propice au développement anarchique de la flore bactérienne saprophyte telle que P. *acnes* et provoquer des lésions acnéiques.

Pour lutter contre l'hyperséborrhée, il a donc été proposé dans l'art antérieur divers composés qui, par application topique sur la peau, sont susceptibles de diminuer la lipogenèse au niveau des sébocytes et limiter, par voie de conséquence, la production de sébum.

Parmi ces composés, on peut citer notamment un extrait de grains de café (FR-2 848 447) et l'acide ascorbique et ses dérivés (EP-0 771 557).

Il est par ailleurs courant d'introduire, dans les produits cosmétiques destinés au soin des peaux grasses, des poudres d'origine naturelle ou synthétique destinées à absorber le sébum, parmi lesquelles on peut citer notamment les charges telles que le talc, l'amidon, la silice et les poudres de polyamide (Nylon). La demanderesse a par ailleurs mis en évidence (EP-1 493 433) que des particules poreuses de polyamide ayant un diamètre moyen, en volume, inférieur ou égal à 10 µm pouvaient être chargées d'actifs destinés notamment au soin des peaux grasses et/ou de dérivés de vitamine C, et permettre ainsi de véhiculer ces actifs dans le follicule pilo-sébacé, sur leur site d'action. Ces compositions peuvent comprendre de l'acide ascorbique en plus des particules chargées. L'Exemple 7 divulgue ainsi une émulsion E/H renfermant des particules de nylon chargées de triclosan, ainsi que de l'acide ascorbique.

Il subsiste toutefois le besoin de disposer de compositions permettant de lutter plus efficacement contre les désordres esthétiques des peaux grasses que celles proposées à ce jour dans l'art antérieur.

La Demanderesse a découvert que ce besoin pouvait être satisfait en associant aux particules poreuses précitées, chargées ou non d'actif(s), un dérivé d'acide ascorbique susceptible de freiner la production de sébum, dans une composition ne se trouvant pas sous la forme d'une émulsion E/H. La composition ainsi obtenue permet d'améliorer la matité de la peau de façon rémanente. En effet, le dérivé d'acide ascorbique limite la production de sébum tandis que les particules poreuses permettent d'absorber le sébum à la surface de la peau et, pour celles ayant pénétré dans les pores par massage de la composition sur la peau, de "pomper" le sébum à sa source, voire de libérer un actif anti-séborrhéique sur son site d'action. La peau se trouve ainsi efficacement matifiée.

On connaît, certes, de la demande WO 00/62789 une crème anti-âge comprenant un glucoside d'ascorbyle et du Nylon-6. Toutefois, les peaux âgées se caractérisent, au contraire des peaux grasses, par une réduction de la production de sébum, qui est surtout marquée après la ménopause et conduit généralement chez les peaux dites matures ou oligoséborrhéiques, à un taux de sébum inférieur à 100 µg/cm² au niveau du front.

La présente invention a donc pour objet l'utilisation cosmétique pour le soin des peaux grasses d'une composition renfermant, dans un milieu physiologiquement acceptable : (a) au moins un dérivé hydrosoluble d'acide ascorbique et (b) des particules poreuses de polyamide ayant un diamètre moyen, en volume, inférieur ou égal à 10 µm, étant entendu que la composition ne se trouve pas sous la forme d'une émulsion eau-dans-huile.

Elle se rapporte également à un procédé cosmétique de soin d'une peau grasse, en particulier en vue de matifier la peau, comprenant l'application topique sur celle-ci d'une composition telle que définie ci-dessus.

Par "matifier", on entend, dans la présente demande, rendre la peau visiblement plus mate, moins brillante. L'effet matifiant de la composition peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

Par "peau grasse", on entend une peau luisante, qui présente en particulier un taux de sébum supérieur à 200 µg/cm² mesuré au niveau du front.

Par "dérivé hydrosoluble d'acide ascorbique", on entend des composés susceptibles de se dissoudre au moins partiellement dans l'eau, qui peuvent notamment être choisis parmi : l'acide ascorbique, un sel métallique d'acide ascorbique phosphorylé et un glucoside d'ascorbyle.

Comme sels métalliques d'acide ascorbique phosphorylé, on peut citer l'ascorbyl phosphate de magnésium et l'ascorbyl phosphate de sodium.

Par "glucoside d'ascorbyle", on entend le produit de condensation du glucose, sous forme D, c'est-à-dire sous forme de glucopyrannose α ou β ou de furannose α ou β, ou sous forme L, avec l'acide ascorbique, de préférence sous forme L. Le 2-O-α-D-glucopyranoside d'acide L-ascorbique est préféré pour une utilisation dans la présente invention. Il est notamment disponible auprès de la société HAYASHIBARA.

La quantité de glucoside d'ascorbyle utilisé selon l'invention peut représenter de 0,01 à 10%, et de préférence de 0,01 à 5% du poids total de la composition.

Par l'expression « particules poreuses », on désigne des particules ayant une structure comportant des pores. Bien que ce mode de réalisation ne soit que facultatif, cette structure poreuse peut permettre au moins en partie l'incorporation d'un ou plusieurs agents actifs au sein desdites particules. Au sens de la présente invention, on utilisera ci-après l'expression « particules chargées » pour désigner les particules selon l'invention qui renferment un actif.

La structure des particules peut être de type matriciel à l'image d'une éponge. Elle peut également être de type vésiculaire, c'est-à-dire que la particule présente une cavité interne délimitée par une paroi poreuse. La porosité associée à la taille des particules est caractérisée quantitativement par leur surface spécifique.

Les particules poreuses de l'invention présentent une surface spécifique mesurée selon la méthode BET supérieure ou égale à 1 m²/g. La méthode BET (BRUNAUER-EMMET-TELLER) est une méthode bien connue de l'homme du métier. Elle est notamment décrite dans « The journal of the American Chemical Society », vol.60, page 309, février 1938 et correspond à la norme internationale ISO 5794/1 (annexe D). La surface spécifique mesurée selon la méthode BET correspond à la surface spécifique totale, c'est-à-dire qu'elle inclue la surface formée par les pores.

Selon un mode de réalisation particulier, les particules de l'invention présentent une surface spécifique mesurée par BET, allant notamment de 2,5 à 100 m²/g.

Les particules poreuses selon l'invention sont généralement des particules individualisées. Par l'expression « particules individualisées », on désigne des particules qui ne sont pas regroupées sous la forme d'agrégat ou d'agglomérat. Ces particules présentent en particulier une masse volumique supérieure ou égale à 0,15 g/cm³ et notamment allant de 0,2 à 5 g/cm³.

Comme mentionné précédemment, les particules selon la présente invention ont un diamètre moyen en volume inférieur ou égal à 10 µm. En effet, de telles particules peuvent pénétrer dans le follicule sébacé par application d'une force mécanique. Cette force mécanique provient généralement d'un massage qui, outre la poussée qu'elle exerce, génère un effet de pompe au niveau du follicule. Les particules atteignent ainsi progressivement le canal folliculaire dans lequel elles sont susceptibles d'absorber le sébum et, le cas échéant, de libérer le composé actif qu'elles portent. Le matériau constitutif des particules est ensuite rejeté grâce à l'écoulement de sébum et/ou à la pousse du poil permettant ainsi d'éviter toute réaction indésirable éventuelle de l'organisme vis-à-vis de ce matériau.

Il faut noter que des particules ayant un diamètre supérieur à 10 µm, même avec application d'une force mécanique similaire, restent pour la plupart localisées sur la surface de la peau sans y pénétrer.

Selon un mode de réalisation particulier de l'invention, les particules ont un diamètre moyen en volume supérieur ou égal à 0,1 µm et en particulier allant de 0,5 à 8 µm. Les particules utilisées selon l'invention sont des particules, notamment sphériques, poreuses, de diamètre moyen en nombre pouvant aller de 0,1 à 50 µm, notamment de 0,1 à 20 µm et tout particulièrement de 0,5 à 10 µm.

Selon une variante de l'invention, les particules se caractérisent par leur homogénéité granulométrique. En particulier, elles présentent un indice de polydispersité, IP, allant de 1 à 4, et notamment inférieur ou égal à 3. Cet indice de polydispersité est défini comme le rapport D(4,3)/D(3,2), dans lequel D(4,3) désigne le diamètre moyen en volume et D(3,2) désigne le diamètre moyen en surface. Ces deux valeurs sont communément mesurées à l'aide de granulomètres à diffraction laser tel que celui commercialisé sous la dénomination de « Mastersizer 2000 » par la société MALVERN.

Les particules poreuses de l'invention peuvent avoir des formes variées, notamment une forme globulaire et en particulier sensiblement sphérique.

Comme particules poreuses selon l'invention, on utilise des particules de polyamide, en particulier de Nylon 6, Nylon 6-6, Nylon 12 ou Nylon 6-12 telles que celles commercialisées par la société ATOFINA sous le nom générique d'« Orgasol ».

Parmi ces composés, on préfère tout particulièrement le Nylon-12.

Les particules poreuses utilisées selon l'invention peuvent être chargées d'au moins un actif de soin des peaux grasses, ou non chargées (vides).

Lorsqu'on utilise selon l'invention des particules chargées, celles-ci peuvent être préparées selon des méthodes conventionnelles, en particulier par imprégnation. En particulier, les particules chargées utilisées selon l'invention peuvent être obtenues par imprégnation de particules poreuses préformées avec au moins un composé actif. Avantageusement, ce protocole ne requiert pas la présence d'un porogène.

A titre d'exemple, les particules poreuses renfermant l'actif sont susceptibles d'être obtenues selon un procédé d'imprégnation comprenant les étapes suivantes :
- la solubilisation de l'actif à encapsuler dans un solvant tel que l'acétone ou l'éthanol, pour obtenir une solution d'imprégnation,
- l'imprégnation des particules poreuses par ladite solution d'imprégnation,
- l'évaporation du solvant jusqu'à l'obtention d'une poudre sèche.

La poudre ainsi obtenue ne contient généralement qu'une très faible proportion de solvant résiduel, de l'ordre de 1/10 ppm.

Comme solvants pouvant être utilisés dans un tel procédé d'imprégnation, on peut citer notamment l'acétone, l'éthanol, l'isopropanol, le dichlorométhane, l'acétate d'éthyle. Bien entendu, le choix du solvant est effectué en tenant compte de la nature des composés à encapsuler.

Généralement, la composition contient de 0,1 à 50 % en poids, de préférence de 0,1 à 20 % en poids, et plus préférentiellement de 0,5 à 5% en poids de particules poreuses (chargées ou non chargées), par rapport au poids total de la composition.

Lorsque ces particules sont chargées, elles contiennent un actif de soin des peaux grasses.

Par l'expression « actif de soin des peaux grasses », on entend, dans le cadre de la présente invention, un composé qui a par lui-même, c'est-à-dire ne nécessitant pas l'intervention d'un agent extérieur pour l'activer, une activité biologique qui peut être en particulier :
- une activité desquamante (qui permet l'ouverture des comédons), et/ou
- une activité anti-microbienne (notamment sur *P. acnes*), et/ou
- une activité anti-inflammatoire, et/ou
- une activité sébo-régulatrice, et/ou
- une activité anti-oxydante (qui empêche l'oxydation du squalène et la formation des comédons).

L'actif de soin des peaux grasses peut donc être choisi parmi : les agents desquamants et/ou anti-microbiens et/ou anti-inflammatoires et/ou sébo-régulateurs et/ou anti-oxydants.

### 1. Agents desquamants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

L'acide n-octanoyl-5-salicylique est préféré pour une utilisation dans la présente invention.

### 2. Agents anti-microbiens

Les agents anti-microbiens susceptibles d'être utilisés dans la composition selon l'invention peuvent notamment être choisis parmi le 2,4,4'-trichloro-2'-hydroxy diphényl éther (ou triclosan), le 3,4,4'-trichlorocarbanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octopirox ou piroctone olamine, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le pidolate de cuivre, l'acide salicylique, l'iodopropynyl butylcarbamate, le farnesol, les phytosphingosines et leurs mélanges.

Les agents antimicrobiens préférés sont l'octopirox, l'acide salicylique et l'iodopropynyl butylcarbamate.

### 3. Agents anti-inflammatoires

Comme agents anti-inflammatoires ou apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, le bisabolol, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, les extraits de camomille, l'allantoïne, le Sépivital EPC (diesterphosphorique de vitamine E et C) de Seppic, les huiles insaturées en oméga 3 telles que les huiles de rosier muscat, de cassis, d'Echium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, les extraits de Centella asiatica, le piperonal, un extrait de clou de girofle, un extrait d'Epilobium Angustifolium, l'aloe vera, un extrait de Bacopa monieri, les phytostérols, la niacinamide, la cortisone, l'hydrocortisone, l'indométhacine et la beta méthasone.

Les agents anti-inflammatoires préférés pour une utilisation dans la présente invention sont les extraits de Centella asiatica, l'acide β-glycyrrhétinique et ses sels, l'alpha-bisabolol et la niacinamide.

### 4. Agents sébo-régulateurs

Lorsque la composition selon l'invention comprend un agent sébo-régulateur tel qu'un inhibiteur de 5α-réductase, celui-ci peut notamment être choisi parmi :
- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5® ;
- un extrait de Laminaria saccharina commercialisé notamment par la société SECMA sous la dénomination commerciale Phlorogine® ;
- un extrait de Spiraea ulmaria commercialisé notamment par la société SILAB sous la dénomination commerciale Sebonormine® ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED;
- des extraits de plantes du genre Silybum ;
- des extraits végétaux contenant des sapogénines et en particulier les extraits de Dioscorées riches en diosgénine ; et
- des extraits d'Eugenia caryophyllata contenant de l'eugénol et du glucoside d'eugényle.

Les sels de zinc sont préférés pour une utilisation dans la présente invention.

### 5. Agents anti-oxydants

Les agents anti-oxydants préférés pour une utilisation dans la présente invention peuvent être choisis parmi le tocophérol et ses esters, tels que l'acétate de tocophérol ; le BHT et le BHA.

Le ou les actifs utilisés dans la composition selon l'invention peuvent représenter de 1 à 50%, de préférence de 2 à 40% et, mieux, de 5 à 30% du poids total des particules chargées.

La composition selon l'invention est généralement adaptée à une application topique sur la peau -généralement la peau du visage- et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et ne génère pas d'inconforts inacceptables (picotements, tiraillements, rougeurs), susceptible de détourner le consommateur ou la consommatrice d'utiliser cette composition.

Cette composition peut être utilisée comme composition de soin, c'est-à-dire non rincée, ou comme composition de nettoyage / démaquillage, c'est-à-dire rincée.

La composition selon l'invention peut se présenter sous toutes les formes galéniques classiquement utilisées pour une application topique, autres qu'une émulsion E/H, et notamment sous forme de dispersions du type lotion ou gel, d'émulsions de consistance liquide ou semi-liquide du type lait, obtenues par dispersion d'une phase grasse dans une phase aqueuse (H/E), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation, la composition utilisée selon l'invention renferme de l'eau et elle se présente avantageusement sous forme d'une émulsion H/E. La composition est en effet de préférence appliquée sur des personnes présentant une peau grasse et il est donc souhaitable qu'elle soit formulée de façon à ce que sa texture soit la plus légère, la moins grasse possible.

Dans le cas où elle se trouve sous la forme d'une émulsion H/E, la composition selon l'invention peut contenir, comme tensioactifs, au moins un composé choisi parmi : les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénées et/ou oxypropylénées, tels que les esters de glycéryle et d'acide gras en C₈-C₂₄, et leurs dérivés oxyalkylénés, les esters de sorbitol et d'acide gras en C₈-C₂₄, et leurs dérivés oxyalkylénés, les esters de sucre (sucrose, glucose, alkylglucose) et d'acide gras en C₈-C₂₄, et leurs dérivés oxyalkylénés ; les esters de polyéthylène glycol et d'acide gras en C₈-C₂₄, et leurs dérivés oxyalkylénés ; les éthers de polyalkylène glycol et d'alcools gras en C₈-C₂₄ les éthers de sucre et d'alcools gras en C₈-C₂₄, et leurs mélanges.

En variante, la composition selon l'invention sous forme d'émulsion peut renfermer un polymère amphiphile ionique et être exempte d'émulsionnant.

Cette composition peut en outre contenir divers adjuvants couramment utilisés dans le domaine cosmétique, tels que des polyols ; des huiles siliconées, volatiles ou non, et/ou hydrocarbonées et/ou des esters d'acides gras ; des alcools gras ; des charges ; des conservateurs ; des séquestrants ; des colorants ; des parfums ; et des épaississants et gélifiants, en particulier les polysaccharides tels que la gomme de xanthane, les homo- et copolymères d'acrylamide, les homo- et copolymères d'acide acrylamido méthylpropane sulfonique (AMPS) et les homo- et copolymères acryliques éventuellement réticulés ; et des ajusteurs de pH.

Bien entendu, l'homme du métier veillera à choisir ce ou ces éventuels composés additionnels et/ou leur quantité de manière telle que les propriétés avantageuses de la composition selon l'invention ne soient pas ou substantiellement pas altérées par l'adjonction envisagée.

Comme charges, on peut citer par exemple, les fibres de polyamide, les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinique, commercialisées sous la dénomination DRY-FLO par la société National Starch ; la silice ; les oxydes métalliques tels que le dioxyde de titane ou l'oxyde de zinc ; le mica ; et leurs mélanges.

La quantité de charge(s) peut aller par exemple de 0,05 à 20 % en poids et mieux 0,1 à 10 % en poids par rapport au poids total de la composition.

La composition utilisée selon l'invention peut aussi comprendre, en plus des particules poreuses (chargées ou non) et du dérivé d'acide ascorbique, des actifs de soin des peaux grasses, choisis dans les listes indiquées précédemment, qui peuvent être identiques ou différents des actifs éventuellement inclus dans les particules chargées. Il peut notamment s'agir de l'acide salicylique et de ses dérivés.

La composition selon l'invention a généralement un pH allant de 3 à 9, de préférence de 4 à 7, mieux, de 4 à 6.

Elle est généralement appliquée sur la peau du visage et éventuellement du cou par massage léger.

L'invention sera maintenant illustrée par les exemples non limitatifs suivants. Dans ces exemples, les quantités sont indiquées en pourcentage pondéral.

### EXEMPLES

### Exemple 1 : Fluide pour peaux grasses comprenant des particules non chargées

On prépare une émulsion H/E ayant la composition ci-dessous, de façon classique pour l'homme du métier.

### Phase A

| | |
|---|---|
| Eau | 61 % |
| Glycérine | 5 % |
| Conservateurs | 0,5 % |

### Phase B

| | |
|---|---|
| Mélange de stéarate de glycéryle et de stéarate de polyoxyéthylène (100 OE) | 2 % |
| Alcool cétylique | 1 % |
| Mono-laurate de sorbitane oxyéthyléné (20 OE) | 2 % |
| Conservateurs | 0,2 % |
| Isononanoate d'isononyle | 6 % |
| Octyldodécanol | 4 % |
| Acide n-octanoyl-5-salicylique | 0,5 % |

### Phase C

| | |
|---|---|
| Cyclohexasiloxane | 5 % |
| Gomme de xanthane | 0,2 % |
| Copolymère acrylique réticulé | 0,45% |

### Phase D

| | | |
|---|---|---|
| Eau | qsp | 100 % |
| Hydroxyde de sodium | qsp | ph 5 |

### Phase E

| | |
|---|---|
| Eau | 3 % |
| 2-O-α-D-glucopyranoside d'acide L-ascorbique | 0,5 % |
| Acide citrique | 0,17 % |

### Phase F

| | |
|---|---|
| Particules de Nylon-12 | 3 % |

### Phase G

| | |
|---|---|
| Amidon | 3 % |

Mode opératoire : la phase A est chauffée sous agitation à 80°C jusqu'à parfaite solubilisation, puis la température est ramenée à 70°C. La phase B est chauffée sous agitation à 70°C jusqu'à l'obtention d'une phase limpide, puis ajoutée à la phase A sous agitation. Les constituants de la phase C sont mélangés à température ambiante, puis la phase C est ajoutée au mélange des phase A et B. Les constituants de la phase D sont mélangés à température ambiante, puis ajoutés au mélange des phases A, B et C. L'ensemble est ensuite refroidi à 25°C. Les constituants de la phase E sont mélangés à température ambiante, puis la phase E est ajoutée au mélange obtenu précédemment. Les phases F et G sont successivement dispersées dans ce mélange.

### Exemple 2 : Fluide pour peaux grasses comprenant des particules chargées

### a) Préparation des particules poreuses chargées

0,5 g de tocophérol et 1 g d'acide n-octanoyl-5-salicylique sont solubilisés dans l'acétone. Dans ce mélange, on introduit 13,5 g de particules poreuses de Nylon-12 commercialisées sous la dénomination "ORGASOL 2002 UD NAT COS" par la société ATOFINA. La dispersion est ensuite introduite dans un évaporateur rotatif afin d'éliminer l'acétone. On obtient alors une poudre chargée en tocophérol et dérivé d'acide salicylique.

### b) Préparation de la composition cosmétique

On prépare une émulsion H/E ayant la composition ci-dessous, de façon classique pour l'homme du métier.

| | | |
|---|---|---|
| **A** - | Eau | 61 % |
| | Glycérine | 5 % |
| | Conservateurs | 0.5 % |
| **B** - | Stéarate de glycéryle et de polyoxyéthylène (100 OE) | 2 % |
| | Alcool cétylique | 1 % |
| | Polysorbate-20 | 2 % |
| | Conservateurs | 0.2 % |
| | Isononanoate d'isononyle | 6 % |
| | Octyldodécanol | 4 % |
| | Acide n-octanoyl-5-salicylique | 0.5 % |
| **C** - | Cyclohexasiloxane | 5 % |
| | Gomme de xanthane | 0.2 % |
| | Copolymère acrylique (Pemulen TR2 de NOVEON) | 0.45 % |
| **D** - | Eau Qsp | 100 % |
| | Soude Qsp | pH 5 |
| **E** | Eau | 3 % |
| | Ascorbyl phosphate de magnésium | 0,3 % |
| | Acide citrique | 0,17 % |
| **F** - | Particules de Nylon chargées | 3 % |

### Mode Opératoire

La phase A est chauffée sous agitation à 80°C jusqu'à parfaite solubilisation puis la température est ramenée à 70°C. La phase B est chauffée sous agitation à 70°C jusqu'à obtention d'une phase limpide puis ajoutée à la phase A sous agitation. La gomme de xanthane et le copolymère acrylique sont dispersés dans le cyclohexasiloxane à température ambiante pendant 10 minutes et la phase C est ajoutée au mélange A + B. Puis la phase D, préalablement solubilisée est ajoutée au mélange ainsi obtenu. Le mélange A+B+C+D est ensuite refroidi à 25°C. Les constituants de la phase E sont mélangés à température ambiante, puis la phase E est ajoutée au mélange obtenu précédemment. La phase F est ensuite ajoutée.

Cette composition peut être appliquée matin et/ou soir sur le visage pour matifier les peaux grasses à tendance acnéique.

### Exemple 3 : Evaluation de l'efficacité de la composition selon l'invention

On a comparé l'efficacité in vivo de deux fluides ayant la composition indiquée dans le Tableau 1 ci-dessous.

**TABLEAU 1**

| Ingrédient | Composition A selon l'invention | Composition témoin B |
|---|---|---|
| **2-O-α-D-glucopyranoside d'acide L-ascorbique** | **0.2 %** | **0 %** |
| Sel de zinc | 0.1 % | 0.002 % |
| Tocophérol | 0.03 % | 0 % |
| Autres actifs* | 0 % | 0.16 % |
| **Particules de Nylon-12** | **0.9 %** | **0 %** |
| Fibres de Nylon-66 | 3 % | 2% |
| Hydroxyacides | 0.67 % | 0.33 % |
| Charges | 4 % | 8 % |
| Emollients / corps gras | 16 % | 2 % |
| Polyols | 7 % | 5 % |
| Ajusteurs de pH | 0.22 % | 1.4 % |
| Alcool | 0 % | 25 % |
| Conservateurs | 1.2 % | 0,02 % |
| Epaississants / gélifiants | 0.55 % | 1.4 % |
| Tensioactifs | 5 % | 0 % |
| Parfum | 0.4 % | 0.5 % |
| Eau | qsp 100 % | qsp 100 % |

| | | |
|---|---|---|
| * n'ayant pas d'effet promoteur de la production de sébum | | |

Ces compositions ont été testées sur des sujets hommes ou femmes agés de 18 à 35 ans ayant la peau grasse (score Sebutape > 2). Les compositions A et B ont été appliquées pendant 4 semaines à raison de deux applications quotidiennes.

Le scorage clinique des pores a été effectué au Dermascore avant traitement et après 4 semaines de traitement. Des Sébutapes ont également été prélevés et soumis à une analyse d'image.

### Résultats :

Pour les sujets ayant appliqué la composition A, le score au bout de 4 semaines de traitement était de 1.93 ± 1.03 (p < 0.001), alors que ce score était de 2.88 ± 1.08 avant traitement, soit une amélioration de 33.1 %. La texture de la peau est significativement améliorée et les pores sont moins visibles. Au contraire, on ne note pas d'amélioration significative du resserrement des pores pour les sujets ayant appliqué la composition B.

En outre, pour les sujets ayant appliqué la composition A, l'analyse d'image des Sébutapes a montré une diminution de 77.8 % du nombre de glandes sébacées actives après 4 semaines de traitement, par rapport au nombre de glandes présentes avant traitement. Parallèlement, la surface totale occupée par les glandes sébacées était réduite de 83.8 % et le taux d'occupation des glandes sébacées était réduit de 83.7 %. Ces résultats montrent donc une diminution significative de la sécrétion sébacée après application de la composition A. Le nombre de glandes sébacées n'était réduit que de 11.8 % pour les sujets ayant appliqué la composition B.

Ainsi, la composition A, qui ne diffère essentiellement de la composition B que par la présence d'un glucoside d'ascorbyle et de particules de polyamide, diminue nettement la sécrétion sébacée par rapport à la composition B .

### Exemple 4 : Mise en évidence de l'activité sébomodératrice de différents dérivés d'acide ascorbique

Différents dérivés d'acide ascorbique ont été testés sur un modèle de sébocytes humains immortalisés en culture, issus de la lignée SZ95 décrite dans Zouboulis, C.C., Seltmann, H., Neitzel, H. & Orfanos, C.E., Establishment and Characterization of an Immortalized Human Sebaceous Gland Cell Line, J. Invest. Dermatol., 113, 1011-1020 (1999).

Ces dérivés étaient constitués : du 2-O-α-D-glucopyranoside d'acide L-ascorbique (ci-après, glucoside d'ascorbyle), de l'acide ascorbique, du palmitate d'ascorbyle et de l'ascorbyl phosphate de magnésium.

Le test a consisté à mesurer la quantité de lipides produite par les sébocytes de la lignée (à confluence), en présence ou non d'agents actifs dilués dans le milieu de culture. Après 2 jours de traitement, les cellules adhérentes sont traitées par du Rouge de Nile (1µg/ml). Le contenu en lipides est ensuite quantifié par mesure de la fluorescence du colorant (deux couples d'excitation/émission : 485-540nm pour les lipides neutres et 540-620 nm pour les lipides non neutres). Les résultats sont donnés pour les lipides neutres (constitutifs du sébum).

L'expérience est réalisée en octoplicate (produits dosés et témoin) en plaque de 96 puits.

Les résultats sont présentés dans le Tableau 2 ci-après.

**TABLEAU 2 Inhibition de la lipogénèse par les dérivés d'acide ascorbique**

| | | | |
|---|---|---|---|
| | Efficacité (% par rapport au témoin) | | |
| | 0,04% | 0,004% | 0,0004% |
| Acide ascorbique | 73 | 84 | ns |
| Glucoside d'ascorbyle | 91 | 84 | 89 |
| Ascorbyl phosphate de magnésium | 80 | 90 | ns |
| Ascorbyl palmitate | -- | 174 | 143 |

| | | | |
|---|---|---|---|
| ns : non significatif | | | |

Comme il ressort de ce tableau, le glucoside d'ascorbyle réduit la lipogénèse à une concentration efficace qui est dix fois inférieure à celle nécessaire à l'acide ascorbique pour obtenir un niveau d'inhibition comparable. En outre, le palmitate d'ascorbyle a un effet de stimulation (et non d'inhibition) de la lipogénèse, le rendant impropre à une utilisation dans le soin des peaux grasses.

## Revendications

1. Utilisation cosmétique pour le soin des peaux grasses d'une composition renfermant, dans un milieu physiologiquement acceptable : (a) au moins un dérivé hydrosoluble d'acide ascorbique et (b) des particules poreuses de polyamide ayant un diamètre moyen, en volume, inférieur ou égal à 10 µm, étant entendu que la composition ne se trouve pas sous la forme d'une émulsion eau-dans-huile.

2. Utilisation selon la revendication 1, **caractérisée en ce que** le dérivé d'acide ascorbique est choisi parmi : l'acide ascorbique, un sel métallique d'acide ascorbique phosphorylé et un glucoside d'ascorbyle.

3. Utilisation selon la revendication 2, **caractérisée en ce que** le sel métallique d'acide ascorbique phosphorylé est choisi parmi l'ascorbyl phosphate de magnésium et l'ascorbyl phosphate de sodium.

4. Utilisation selon la revendication 2, **caractérisée en ce que** le glucoside d'ascorbyle est le 2-O-α-D-glucopyranoside d'acide L-ascorbique.

5. Utilisation selon l'une quelconque des revendications 1 à 4, **caractérisée en ce que** les particules poreuses ont une surface spécifique mesurée selon la méthode BET supérieure ou égale à 1 m²/g.

6. Utilisation selon l'une quelconque des revendications 1 à 5, **caractérisée en ce que** les particules poreuses ont un diamètre moyen en volume allant de 0,5 à 8 µm.

7. Utilisation selon l'une quelconque des revendications 1 à 6, **caractérisée en ce que** les particules poreuses sont des particules de Nylon 6, Nylon 6-6, Nylon 12 ou Nylon 6-12.

8. Utilisation selon l'une quelconque des revendications 1 à 7, **caractérisée en ce que** les particules poreuses sont chargées d'au moins un actif de soin des peaux grasses.

9. Utilisation selon la revendication 8, **caractérisée en ce que** les particules poreuses sont susceptibles d'être obtenues selon un procédé d'imprégnation comprenant les étapes suivantes :
- la solubilisation de l'actif à encapsuler dans un solvant tel que l'acétone ou l'éthanol, pour obtenir une solution d'imprégnation,
- l'imprégnation des particules poreuses par ladite solution d'imprégnation,
- l'évaporation du solvant jusqu'à l'obtention d'une poudre sèche.

10. Utilisation selon l'une quelconque des revendications 1 à 9, **caractérisée en ce que** la composition contient de 0,5 à 5% en poids de particules poreuses, par rapport au poids total de la composition.

11. Utilisation selon la revendication 8 ou 9, **caractérisée en ce que** l'actif de soin des peaux grasses est choisi parmi les agents desquamants et/ou anti-microbiens et/ou anti-inflammatoires et/ou sébo-régulateurs et/ou anti-oxydants.

12. Utilisation selon la revendication 11, **caractérisée en ce que** l'agent desquamant est l'acide n-octanoyl-5-salicylique.

13. Utilisation selon la revendication 11, **caractérisée en ce que** l'agent anti-microbien est choisi parmi : l'octopirox, l'acide salicylique et l'iodopropynyl butylcarbamate.

14. Utilisation selon la revendication 11, **caractérisée en ce que** l'agent anti-inflammatoire est choisi parmi : les extraits de Centella asiatica, l'acide β-glycyrrhétinique et ses sels, l'alpha-bisabolol et la niacinamide.

15. Utilisation selon la revendication 11, **caractérisée en ce que** l'agent sébo-régulateur est choisi parmi les sels de zinc.

16. Utilisation selon la revendication 11, **caractérisée en ce que** l'agent anti-oxydant est choisi parmi le tocophérol et l'acétate de tocophérol.

17. Utilisation selon l'une quelconque des revendications 8 à 16, **caractérisée en ce que** l'actif représente de 5 à 30% du poids total des particules chargées.

18. Utilisation selon l'une quelconque des revendications 1 à 17, **caractérisée en ce que** la composition se présente sous forme d'une émulsion H/E.

19. Utilisation selon l'une quelconque des revendications 1 à 18, **caractérisée en ce que** la composition a un pH allant de 4 à 6.

20. Procédé cosmétique de soin d'une peau grasse, en particulier en vue de matifier la peau, comprenant l'application topique sur celle-ci d'une composition telle que définie ci-dessus.
